# EUROPÄISCHE PATENTSCHRIFT

(11) **EP 2 421 580 B1**
(45) Veröffentlichungstag und Bekanntmachung des Hinweises auf die Patenterteilung: **04.12.2019**
(21) Anmeldenummer: 10713854.7
(22) Anmeldetag: 14.04.2010
(51) Int. Cl.: A61M 1/36

(54) **AUFNAHMEEINRICHTUNG ZUM AUFNEHMEN VON MEDIZINISCHEN FLUIDEN SOWIE EXTERNE FUNKTIONSEINRICHTUNG UND MEDIZINISCHE BEHANDLUNGSVORRICHTUNG**
RECEIVING APPARATUS FOR RECEIVING MEDICAL FLUIDS AND EXTERNAL FUNCTIONAL APPARATUS AND MEDICAL TREATMENT DEVICE
DISPOSITIF RÉCEPTEUR POUR LA RÉCEPTION DE FLUIDES MÉDICAUX, DISPOSITIF FONCTIONNEL EXTERNE, ET DISPOSITIF DE TRAITEMENT MÉDICAL

(30) Priorität: 23.04.2009 DE 102009018664; 10.06.2009 DE 102009024466; 10.06.2009 US 185607 P
(43) Veröffentlichungstag der Anmeldung: 29.02.2012
(73) Patentinhaber: Fresenius Medical Care Deutschland GmbH, 61352 Bad Homburg v.d.H. (DE)
(72) Erfinder: LAUER, Martin, 66606 St. Wendel (DE); GÜNTHER, Götz, 61440 Oberursel (DE)
(74) Vertreter: Bobbert & Partner Patentanwälte PartmbB
(86) Internationale Anmeldenummer: PCT/EP2010/002294
(87) Internationale Veröffentlichungsnummer: WO 2010/121740

(56) Entgegenhaltungen:
- WO-A1-2007/000066
- US-A- 6 013 060
- US-A1- 2005 230 292
- US-A1- 2009 084 721
- US-B1- 7 279 031

## Beschreibung

Die vorliegende Erfindung betrifft eine Aufnahmeeinrichtung zum Aufnehmen von medizinischen Fluiden gemäß dem Oberbegriff des Anspruchs 1. Sie betrifft ferner eine externe Funktionseinrichtung gemäß Anspruch 14 sowie eine medizinische Behandlungsvorrichtung gemäß Anspruch 15.

Verschiedene Vorrichtungen, wie beispielsweise Behandlungsvorrichtungen der Medizintechnik, weisen Aufnahmeeinrichtungen zum vorübergehenden Aufnehmen von Fluiden auf. Diese werden während des Betriebs der Vorrichtung i.a.R. mehrfach mit Fluid befüllt und auch wieder entleert. Beim Befüllen und Entleeren üblicherweise auftretende Strömungsphänomene werden regelmäßig mittels geeigneter baulicher Maßnahmen beeinflusst. Zu ihnen zählen Behälterwanddüsen, Prallwände sowie geometrische Ausgestaltungen von Bauelementen z.B. in Tauchrohrformen, Diffusorformen und dergleichen.

Die US 7,279,031 B1 offenbart eine Vorrichtung zur Vermeidung von Thrombenbildung. Die US 2009/0084721 A1 offenbart Dialysesysteme mit Luftabscheidern.

Die WO 2007/000066 A1 offenbart ein Verfahren und eine Vorrichtung zum Ablassen und Beseitigen von bestimmtem Material.

Die US 6,013,060 A offenbart eine Vorrichtung zum Regeln von intravenös applizierten Fluiden.

Die US 2005/230292 A1 offenbart eine Vorrichtung zur Behandlung von medizinischen Fluiden, die eine Kassette mit Phantomventilen umfasst.

Eine Aufgabe der vorliegenden Erfindung ist, eine weitere Aufnahmeeinrichtung zum Aufnehmen von medizinischen Fluiden, insbesondere Blut, mit einer Einrichtung zum Beeinflussen einer Fluidströmung anzugeben.

Die erfindungsgemäße Aufgabe wird durch eine Aufnahmeeinrichtung zum Aufnehmen eines oder mehrerer medizinischer Fluide mit den Merkmalen des Anspruchs 1 gelöst.

Die erfindungsgemäße Aufnahmeeinrichtung weist eine Einströmungsöffnung auf, durch welche wenigstens ein medizinisches Fluid in ein Inneres der Aufnahmeeinrichtung einführbar oder einbringbar ist. Sie weist ferner wenigstens ein Fluidschwallumleitungselement zum Umleiten des Fluidschwalls des einströmenden Fluids auf.

Der Begriff "Einströmungsöffnung", wie er hierin verwendet wird, bezeichnet eine Öffnung oder eine Aussparung - z. B. in einer Seite bzw. Seitenfläche bzw. Seitenwand der Aufnahmeeinrichtung - durch welche ein Fluid in das geschlossene oder zur Umgebung offene Innere der Aufnahmeeinrichtung einströmen, eintreten oder einfließen kann.

Ein "Fluid" im Sinne der vorliegenden Erfindung schließt jede medizinische Flüssigkeit und/oder jedes medizinische Gas und/oder jedes Flüssigkeits-Gas-Gemisch (z.B. Gasblasen in Flüssigkeit, Schaum, insbesondere Blut mit Luftblasen, Blutschaum) ein. Insbesondere kann es sich um Blut in einem extrakorporalen Blutkreislauf handeln.

Das "Fluid" ist hier und im Folgenden gleichbedeutend als medizinisches Fluid zu verstehen.

Ein "Inneres der Aufnahmeeinrichtung" bezeichnet ein Volumen bzw. einen Rauminhalt der Aufnahmeeinrichtung, welches zum vollständigen oder teilweise Befüllen mit Fluiden und Aufnehmen derselben geeignet und vorgesehen ist.

Ein "Fluidschwallumleitungselement" bezeichnet einen Körper, welcher zum Beeinflussen eines Fluidschwalls, insbesondere zum Umleiten eines auf ihn auftreffenden Fluidschwalls, geeignet ist. Dieser Körper kann ein um- und/oder überströmbares Element sein.

Dabei kann das Fluidschwallumleitungselement erfindungsgemäß auch als ein Fluidkontaktelement verstanden werden, welches in der Aufnahmeeinrichtung mit dem Ziel vorgesehen ist, von dem in das Innere der Aufnahmeeinrichtung einströmenden Fluidschwall getroffen zu werden bzw. mit diesem in Kontakt zu treten. Dieser Kontakt ist konstruktiv absichtlich herbeigeführt, damit der Fluidschwall auf beabsichtigte Weise beeinflusst wird. Dies kann ein Abschwächen des Impulses des Fluidschwalls bedeuten, ein Auslöschen des Impulses des Fluidschwalls im Sinne einer Übertragung. Es kann ferner insbesondere ein gezieltes Aufteilen des Fluidschwalls in wenigstens zwei nachverfolgbare Fluidteilschwalle oder viele kleine Fluidteilschwalle bedeuten, wie unten stehend detaillierter ausgeführt wird.

Das Fluidschwallumleitungselement weist hierzu erfindungsgemäß wenigstens einen Umströmungskörper auf, welcher ausgelegt und/oder vorgesehen und geeignet ist, den Fluidschwall in wenigstens zwei Teilströme aufzuteilen. Die Teilströme liegen nach ihrer Aufteilung - zumindest vorübergehend - körperlich getrennt voneinander, also ohne Kontakt zueinander, vor.

Der Begriff "Fluidschwall" bezeichnet eine Menge oder ein Volumen des in die Aufnahmeeinrichtung eingebrachten oder einströmenden Fluids.

Der Fluidschwall kann ein kontinuierlicher und/oder konstanter (z.B. mit einer konstanten Geschwindigkeit einströmender) Fluidschwall oder ein zu einem bestimmten Zeitpunkt und/oder nur über einen begrenzten, bestimmten Zeitraum in die Aufnahmeeinrichtung einströmender Fluidschwall sein.

Der Fluidschwall kann einen Impuls übertragen. Der Fluidschwall kann dergestalt sein, dass sein Einströmen in die Aufnahmeeinrichtung ohne Vorsehen eines wie oben beschriebenen Fluidschwallumleitungselements zu Turbulenzen und/oder zu Gaseinschlüssen (insbesondere von Luft) in das einströmende Fluid (insbesondere eine Flüssigkeit) oder in einem Fluid, in das ein weiteres Fluid einströmen soll (insbesondere Blut) führen würde.

Der Begriff "Teilfluidschwall" bezeichnet einen durch Aufteilen des ursprünglich in die Aufnahmeeinrichtung einströmenden Fluidschwalls mittels des Umströmungskörpers erhaltenen Teilstrom oder ein Teilvolumen des ursprünglich eingeströmten gesamten Fluidschwalls. Der Teilfluidschwall ist ein Anteil des Fluidschwalls.

Mittels der Aufteilung des Fluidschwalls in Teilfluidschwalle kann das Auftreten unerwünschter Strömungsphänomene vorteilhaft verhindert werden.

Vorteilhafte Weiterentwicklungen der vorliegenden Erfindung sind jeweils Gegenstand der Unteransprüche.

Die Gesamtheit aller Anteile der mittels des Umströmungskörpers erhalten Teilfluidschwalle, welche nach Aufteilung des Fluidschwalls in die Teilfluidschwalle weiter in das Innere der Aufnahmeeinrichtung verbleiben, können sich in einer bevorzugten Ausführungsform der Erfindung im Wesentlichen zu 100 % des gesamten Fluidschwalls addieren. Dies bedeutet, dass alle Teilfluidschwalle weiterhin in die Aufnahmeeinrichtung eintreten oder zunächst in dieser verbleiben.

Die Anteile des Strömungsvolumens oder der Strömungsmenge des einzelnen Teilfluidschwalls können jeweils gleich oder verschieden voneinander sein.

In der Erfindung ist der Umströmungskörper in einem Einströmungsbereich des Fluidschwalls angeordnet, und im Ausströmungsbereich eines Phantomventils in einer Blutkassette zur extrakorporalen Blutbehandlung.

Der Begriff "Phantomventil", wie er hierin verwendet wird, bezeichnet ein Element mit einer mittels Aktor erreichbaren Aktor-Fläche (hier beispielsweise eine Aktor-Membran), welches die Funktion eines Ventils übernehmen kann.

Die Aktor-Membran ist unter Aufbringung einer Kraft hierauf, z.B. einer Druckkraft, in eine Richtung bewegbar, ausdehnbar bzw. wölbbar oder dergleichen. Durch das Bewegen oder Ausdehnen der Aktor-Membran kann sich diese an ein Element, wie eine Abdichtungseinrichtung, etwa einen Steg, anlegen oder von diesem entfernen. Die Aktor-Membran kann somit beispielsweise eine Abdichtung bewirken bzw. verstärken oder beenden bzw. verringern.

Wenn die Kraft auf die Aktor-Membran aufgehoben wird, kann diese in beispielsweise eine Grundposition, z.B. einen nicht gewölbten Zustand, zurückkehren.

Der Begriff "Einströmungsbereich" bezeichnet den Bereich der Aufnahmeeinrichtung, in welchem sich der Fluidschwall unmittelbar nach Einströmen oder Einfließen in die Aufnahmeeinrichtung befindet. Der Einströmungsbereich kann dabei einen aufnahmeeinrichtungsseitigen Bereich um die Einströmungsöffnung herum in räumlichem Bezug zu dieser ausbilden, in welchem sich das durch die Einströmungsöffnung einströmende Fluid in der Aufnahmeeinrichtung auszubreiten und die Aufnahmeeinrichtung zu füllen beginnt.

In einer weiteren bevorzugten Ausführungsform ist der Umströmungskörper zentral (oder in einem zentralen Bereich) im Einströmungsbereich des Fluids, bezogen auf eine Mittelachse der Einströmungsöffnung oder der Einströmungsrichtung, angeordnet.

Die "Mittelachse" bezeichnet dabei z.B. diejenige Achse, die auf einem mittleren Bereich oder einem Mittelpunkt des Querschnitts der Einströmungsöffnung oder der Einströmungsrichtung, wie beispielsweise dem Kreismittelpunkt einer Einströmungsöffnung mit kreisförmigem Querschnitt, senkrecht steht. Die Mittelachse kann parallel zu einer Hauptströmungseinrichtung des gesamten Fluidschwalls vor dessen Aufteilung in Teilfluidschwalle verlaufen.

Die Begriffe "zentral" und "mittig" werden in der vorliegenden Erfindung gleichbedeutend bzw. austauschbar verwendet. Sie sollen jeweils eine Anordnung einer Komponente in einem mittleren Bereich bzw. der Mitte eines Bezugssystems bzw. einer Bezugskomponente bezeichnen. Dabei ist eine zentrale oder mittige Anordnung bevorzugt derart definiert, dass der Formmittelpunkt, z.B. der Kreismittelpunkts des Querschnitts einer Komponente, auf einer Mittelachse oder einem Mittelpunkt der Bezugskomponente angeordnet ist. Formmittelpunkt, Mittelachse oder Mittelpunkt können wie allgemein üblich geometrisch bestimmt oder näherungsweise ermittelt werden.

Der Begriff "Querschnitt" kann, wie vorliegend verwendet, einen Querschnitt senkrecht zu einer Einströmungsrichtung oder parallel zu dieser verstanden werden. Beim "Querschnitt" kann es sich im Rahmen der Diskussion der vorliegenden Erfindung ungeachtet weiterer Merkmale desselben vorzugsweise um den Querschnitt maximalen Durchmessers oder maximaler Abmessungen handeln.

Die Einströmungsöffnung kann beispielsweise in einem Flächeninneren oder einer Mitte einer Seite bzw. Seitenfläche bzw. Seitenwand der Aufnahmeeinrichtung angeordnet sein. Sie kann jedoch auch in einem Randbereich der Seite oder einer Begrenzung der Aufnahmeeinrichtung angeordnet sein.

Die Einströmungsöffnung kann sich über eine im Wesentlichen gesamte Länge einer Seite der Aufnahmeeinrichtung erstrecken. Die Einströmungsöffnung kann sich unabhängig hiervon über eine im Wesentlichen gesamte Breite der Seite der Aufnahmeeinrichtung erstrecken. Sie kann daher verschiedene räumliche Ausgestaltungen annehmen.

Vorzugsweise ist die Einströmungsöffnung jedoch im Wesentlichen zentriert in einer Begrenzungsfläche der Aufnahmeeinrichtung vorgesehen, d.h. der Mittelpunkt (beispielsweise der Kreismittelpunkt einer Einströmungsöffnung mit kreisförmigem Querschnitt) der Einströmungsöffnung kann auf halber Länge und/oder halber Breite der Seitenfläche der Aufnahmeeinrichtung angeordnet sein.

Die Einströmungsöffnung kann einen kreisförmigen oder elliptischen Querschnitt aufweisen, sie kann jedoch auch jeden anderen Querschnitt aufweisen, der zum Zwecke der vorliegenden Erfindung geeignet scheint.

Es können auch mehrere gleichartig oder verschiedenartig ausgestaltete Einströmungsöffnungen in der Aufnahmeeinrichtung vorgesehen sein. Die Ausgestaltung derselben kann jeweils strömungsoptimiert und für den angestrebten Zweck geeignet ausgewählt werden.

Falls mehrere Einströmungsöffnungen in einer oder mehreren Seitenwänden oder Seitenflächen der Aufnahmeeinrichtung vorgesehen sind, kann wenigstens eine der Einströmungsöffnungen dabei erfindungsgemäß ein Fluidschwallumleitungselement bzw. einen Umströmungskörper im Einströmungsbereich des Fluidschwalls aufweisen.

Der Umströmungskörper kann bevorzugt symmetrisch ausgestaltet sein. Der Umströmungskörper kann bevorzugt symmetrisch bezogen auf die Mittelachse der Einströmungsöffnung ausgestaltet bzw. angeordnet sein.

Dies umfasst, dass ein Querschnitt des Umströmungskörpers in einer Ebene senkrecht zur Einströmungsrichtung des in die Aufnahmeeinrichtung eingebrachten Fluidschwalls zu der Mittelachse der Einströmungsöffnung symmetrisch angeordnet bzw. vorgesehen sein kann. Bevorzugt ist eine Symmetrie in der näheren Umgebung des Fluidschwallelements vorgesehen. Die gesamte Einrichtung muss hingegen nicht zwingend symmetrisch sein.

Der Umströmungskörper kann allgemein eine Front oder Prallfläche, auf welche der einströmende Fluidschwall oder ein wesentlicher Anteil hiervon auftrifft, und eine Rückseite, an welcher die Teilfluidschwalle zusammengeführt werden können, aufweisen.

Ein erneutes Zusammenführen der aufgeteilten Teilfluidschwalle an einem rückwärtigen Abschnitt des Umströmungskörpers oder einem stromabwärts gelegenen Abschnitt desselben ist von der Erfindung umfasst. Die Erfindung ist jedoch nicht auf ein erneutes Zusammenführen beschränkt; anders formuliert ist dies keine erforderliche Wirkung einer jeden Ausführungsform der Erfindung.

Der Umströmungskörper kann ferner eine linke und eine rechte Seitenflanke aufweisen, an welcher jeweils ein Teilfluidschwall entlang strömen kann.

Ein symmetrisch ausgestalteter Umströmungskörper kann derart ausgestaltet sein, dass die geometrische Form bzw. Ausgestaltung der linken und der rechten Seitenflanke spiegelsymmetrisch zur Mittelachse der Einströmungsöffnung vorgesehen ist.

Die geometrische Form der Front und der Rückseite des Umströmungskörpers können ebenfalls spiegelsymmetrisch zu einer Achse senkrecht zu der Mittelachse der Einströmungsöffnung, d.h. senkrecht zu der Strömungsrichtung des in die Aufnahmeeinrichtung einströmenden Fluidschwalls, angeordnet sein. Die Erfindung ist jedoch nicht hierauf beschränkt.

Beispielsweise kann der Querschnitt des Umströmungskörpers kreisförmig sein, wobei in diesem Fall der Umströmungskörper selbst rotationssymmetrisch ausgestaltet ist.

Alternativ kann der Querschnitt des Umströmungskörpers elliptisch ausgestaltet sein, wobei in diesem Fall jeweils die linke und rechte Seitenflanke des Umströmungskörpers sowie die Front und die Rückseite des Umströmungskörpers zueinander symmetrisch ausgestaltet sind.

Der Querschnitt des Umströmungskörpers kann auch paraboloid oder gotisch spitz oder anders ausgestaltet sein.

Der Querschnitt des Umströmungskörpers kann sich entlang der Höhe oder Tiefe des Umströmungskörpers verändern. Beispielsweise kann die Querschnittsfläche über die Höhe des Umströmungskörpers variieren, z.B. können sich die Querschnittsfläche oder ein Durchmesser derselben über die Höhe verjüngen, d.h. kleiner werden oder abnehmen. Der Querschnitt des Umströmungskörpers kann jedoch entlang der Höhe oder Tiefe des Umströmungskörpers auch konstant bleiben.

Die Querschnittsfläche bzw. der Durchmesser können über die Höhe des Umströmungskörpers mehrfach abnehmen und zunehmen (oder umgekehrt), um ein für die jeweilige Anwendung und das jeweils eingesetzte Fluid optimierte Fluidschwallumleitung zu erreichen.

Der Umströmungskörper kann ausgestaltet sein, um den ursprünglich in die Aufnahmeeinrichtung eingebrachten Fluidschwall in mehr als zwei Teilfluidschwalle aufzuteilen.

Der Umströmungskörper kann beispielsweise in einem zentralen Bereich eine Durchtrittsöffnung zur Durchleitung eines dritten Teilfluidschwalls aufweisen.

Die Durchtrittsöffnung kann sich beispielsweise von der Front bis zur Rückseite des Umströmungskörpers erstrecken.

In einer weiter bevorzugten Ausführungsform ist der Umströmungskörper mit dem 0,4fachen bis 2fachen des Durchmessers der Einströmungsöffnung oder des Strömungskanaldurchmessers von der Einströmungsöffnung beabstandet im Einströmungsbereich der Aufnahmeeinrichtung angeordnet.

Dieser Abstand kann sich vorzugsweise auf eine Entfernung zwischen der Einströmungsöffnung, beispielsweise der Mündung der Einströmungsöffnung in der Seitenfläche oder Seitenwand der Aufnahmeeinrichtung, und der Front (z.B. des am weitesten strömungsaufwärts des Fluidschwalls oder des einen Hauptaufprallpunktes des Fluidschwalls) gelegenen Punktes des Umströmungskörpers beziehen.

In einer weiteren bevorzugten Ausführungsform kann ein Durchmesser des Umströmungskörpers in einer Ebene senkrecht zur Mittelachse oder senkrecht zur Hauptaufprallrichtung des Fluidschwalls auf den Umströmungskörper der Einströmungsöffnung 0,4 bis 1,2-mal so groß wie der Durchmesser der Einströmungsöffnung oder des Strömungskanaldurchmessers sein.

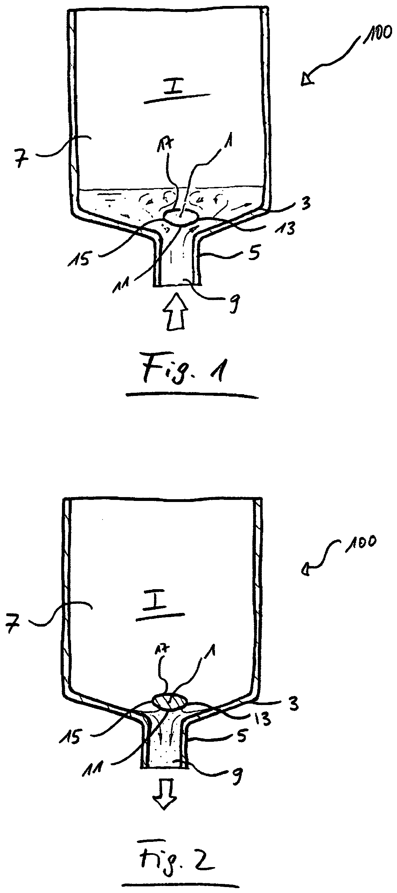

Dieser Durchmesser kann bevorzugt der maximale Durchmesser des Umströmungskörpers sein, beispielsweise der längere Durchmesser eines Umströmungskörpers mit elliptischem Querschnitt, wie er in Fig. 1 gezeigt ist.

In einer weiter bevorzugten Ausführungsform ist das Fluidschwallumleitungselement stoffschlüssig mit der Aufnahmeeinrichtung ausgestaltet.

Das Fluidumschwallumleitungselement bzw. der Umströmungskörper kann während der Herstellung der Aufnahmeeinrichtung gemeinsam mit dieser ausgestaltet bzw. ausgeformt sein. Dies kann beispielsweise mittels Spritzgießen der Aufnahmeeinrichtung erreicht werden. Die Aufnahmeeinrichtung kann beispielsweise als prismatische Spritzgusskammer ausgestaltet sein.

Die Aufnahmeeinrichtung kann in Kassettenbauform oder als Teil einer solchen ausgestaltet sein. Sie kann beispielsweise eine externe Funktionseinrichtung oder Teil einer externen Funktionseinrichtung sein. Die Aufnahmeeinrichtung kann vorzugsweise stoffschlüssig in die externe Funktionseinrichtung integriert sein.

In einer weiter bevorzugten Ausführungsform ist die Aufnahmeeinrichtung an der Einströmungsöffnung mit einem Einströmungskanal verbunden. Sie kann alternativ mit mehreren Einströmungskanälen verbunden sein. Der Anschlusskanal bzw. Einströmungskanal kann einstückig in das untere Ende der Aufnahmeeinrichtung integriert sein.

Der Einströmungskanal kann ein geschlossener, halboffener Kanal oder eine Leitung sein. Er kann in eine externe Funktionseinrichtung integriert sein. Ein solcher Kanal bzw. eine solche Leitung kann während der Herstellung der externen Funktionseinrichtung, beispielsweise mittels Spritzgießen, ausgestaltet bzw. ausgeformt werden.

In einer weiter bevorzugten Ausführungsform kann die Einströmungsöffnung geeignet und vorgesehen sein, in das Innere der Aufnahmeeinrichtung eingeströmte Fluide ausströmen zu lassen.

Dies kann beispielsweise von Vorteil sein, um einen möglichst vollständigen Austausch von Fluiden beim Befüllen oder Entleeren der Aufnahmeeinrichtung zu bewerkstelligen.

Dies kann insbesondere von Vorteil sein, wenn die Aufnahmeeinrichtung Teil einer externen Funktionseinrichtung, wie beispielsweise einer Blutkassette oder Blutbehandlungskassette ist, und eine platzsparende Anordnung der einzelnen Komponenten der Blutkassette wesentlich oder erforderlich oder gewünscht ist.

Zur Einstellung einer Strömungsrichtung der in die Aufnahmeeinrichtung ein- oder ausströmenden Fluide kann die Einströmungs- bzw. Ausströmungsöffnung beispielsweise mit einem oder mehreren verschiedenen und/oder verschiedenartig bzw. unterschiedlich ausgestalteten oder vorgesehenen Vorsprüngen, Einrückungen, Strömungs- bzw. Umströmungselementen und dergleichen in der Öffnung selbst, d.h. in der Durchtrittsfläche derselben, versehen sein.

Zu einer vollständigen Entleerung der Aufnahmeeinrichtung über das Auslaufen der Flüssigkeit nach unten (Schwerkraftentleerung), kann der Ablaufkanal bzw. der Ausströmungskanal am geodätisch tiefsten Punkt der Aufnahmeeinrichtung angeordnet sein.

Die Seitenwände der Aufnahmeeinrichtung können in der Umgebung des Ablaufkanals bzw. des Ausströmungskanals eine gewisse Mindestschräge aufweisen, welche das Ausströmen der in der Aufnahmeeinrichtung vorhandenen Fluide erleichtern können. Somit kann vorteilhaft auch eine nicht vollständig vertikal ausgerichtete Aufnahmeeinrichtung leerlaufen.

Ferner können mit einer solchen Anordnung Strömungstotzonen vorteilhaft verringert oder gar vermieden werden.

In einer weiteren bevorzugten Ausführungsform ist die Aufnahmeeinrichtung in einem extrakorporalen Blutkreislauf angeordnet.

Das Fluidschwallumleitungselement, insbesondere ein Blutschwallumleitungselement oder Blutschwallelement, kann zum Erzielen einer Strömungsverlangsamung, zum Erzeugen einer Turbulenz und/oder Umleiten eines in einer Single-Needle-Kammer befindlichen Blutschwalls oder zum Auslöschen des Impulses des Blutschwalls geeignet und vorgesehen sein. Ein solches Blutschwallumleitungselement kann insbesondere strömungsoptimiert ausgestaltet sein. Es kann beispielsweise in Form einer ellipsoiden oder runden Säule ausgestaltet sein, welche an wenigstens einem Abschnitt ihres Umfangs mit einer Wandung der Single-Needle-Kammer verbunden ist.

Ohne Blutschwallumleitungselement könnte ein durch das Phantomventil einströmender Blutschwall ggf. eine Fontäne bewirken. Dies könnte zu Schwappbewegungen des Flüssigkeitsspiegels und/oder zur Schaumbildung führen. Mittels des Blutschwallelements wird der Gesamtblutschwall in zwei kleinere Blutschwalle aufgeteilt, wodurch der Impuls des Gesamtblutschwalls ausgelöscht werden kann und vorteilhaft Fontänenbildung, Schwappbewegung und/oder Schaumbildung vermieden werden können.

Die erfindungsgemäße Aufgabe wird auch durch eine externe Funktionseinrichtung gemäß Anspruch 14 gelöst. Alle mittels der erfindungsgemäßen Aufnahmeeinrichtung erzielbaren Vorteile lassen sich auch mit der erfindungsgemäßen externen Funktionseinrichtung erreichen.

Eine erfindungsgemäße externe Funktionseinrichtung weist wenigstens eine erfindungsgemäße Aufnahmeeinrichtung auf.

Die erfindungsgemäße externe Funktionseinrichtung kann zur Verwendung in einem Behandlungsverfahren, insbesondere in einem extrakorporalen Blutbehandlungsverfahren, vorgesehen sein. Behandlungsverfahren im Sinne der vorliegenden Erfindung schließen medizinische Behandlungsverfahren - insbesondere Blutbehandlungsverfahren wie Dialyseverfahren, Verfahren der Labortechnik, Verfahren der Nahrungs- oder Arzneimittelherstellung und dergleichen - ein.

In einer bevorzugten Ausführungsform ist die erfindungsgemäße externe Funktionseinrichtung als Blutkassette ausgestaltet.

Die externe Funktionseinrichtung kann zur Verwendung in oder an einer Behandlungsvorrichtung vorgesehen sein.

Die externe Funktionseinrichtung kann an wenigstens einer Seite mit einem Abdeckungselement versehen sein.

Ein "Abdeckungselement" kann beispielsweise eine Membran, eine Folie und dergleichen sein. Beispielhafte Ausführungen für geeignete Abdeckungselemente sowie deren Ausgestaltung und Anordnung an der externen Funktionseinrichtung können beispielsweise der von der Anmelderin der vorliegenden Erfindung beim DPMA am 10. März 2009 eingereichten Anmeldung 10 2009 012 632 A1 mit dem Titel *"Abdichtungseinrichtung zum Abdichten eines Volumens einer medizinischen Behandlungsanordnung gegen ein weiteres Volumen sowie Anordnung und Verfahren"* entnommen werden.

Das Abdeckungselement der externen Funktionseinrichtung kann bevorzugt so angeordnet bzw. vorgesehen sein, dass es an einer Oberseite des Umströmungskörpers aufliegt, insbesondere auf einem auf der Oberseite des Umströmungskörpers umlaufenden Dichtungssteg.

Auf diese Weise kann es möglich sein, eine fluiddichte Abdichtung der externen Funktionseinrichtung gegen ein Äußeres zu erreichen, wobei die Oberseite des Umströmungskörpers einen weiteren Auflagepunkt bereitstellen kann.

Zugleich kann die Abdichtung zwischen der Abdichtungseinrichtung und der Oberseite des Umströmungskörpers eine Abdichtung für das im Inneren der Aufnahmeeinrichtung befindliche Fluid bereitstellen, so dass dieses an der Oberseite der Aufnahmeeinrichtung nicht aus der Aufnahmeeinrichtung heraus in andere Kompartimente der externen Funktionseinrichtung und oder in ein Äußeres der externen Funktionseinrichtung gelangen kann.

Die externe Funktionseinrichtung kann beispielsweise als Blutkassette ausgestaltet sein, wie sie in der von der Anmelderin der vorliegenden Erfindung in den beim DPMA am 23. April 2009 bzw. 10. Juni 2009 eingereichten Anmeldungen 10 2009 018 664 A1 bzw. 10 2009 024 468 A1 mit jeweils dem Titel *"Externe Funktionseinrichtung, Blutbehandlungsvorrichtung zum Aufnehmen einer erfindungsgemäßen externen Funktionseinrichtung, sowie Verfahren"* beschrieben ist.

Eine solche externe Funktionseinrichtung kann eine Einmalkomponente oder ein Einmalartikel sein.

Die externe Funktionseinrichtung kann beispielsweise aus einem Kunststoffmaterial gefertigt sein.

Die externe Funktionseinrichtung kann als Spritzguss-Hartteil mit einer aufgeschweißten Abdeckeinrichtung, z.B. einer Folie, ausgestaltet sein.

Die erfindungsgemäße Aufgabe wird auch durch eine erfindungsgemäße medizinische Behandlungsvorrichtung gemäß Anspruch 15 gelöst. Alle Vorteile, die sich mit der erfindungsgemäßen Aufnahmeeinrichtung erzielen lassen, lassen sich auch mit der erfindungsgemäßen Behandlungsvorrichtung erzielen.

Die erfindungsgemäße Behandlungsvorrichtung kann wenigstens eine erfindungsgemäße Aufnahmeeinrichtung und/oder wenigstens eine erfindungsgemäße externe Funktionseinrichtung aufweisen.

Die Behandlungsvorrichtung kann in einem Behandlungsverfahren, wie es vorstehend angegeben ist, eingesetzt werden.

Beispielsweise kann die Behandlungsvorrichtung eine Blutbehandlungsvorrichtung, wie eine Dialysiervorrichtung, zum Durchführen einer Dialysebehandlung, wie einer Hämodialyse, Hämofiltration, Hämodiafiltration und dergleichen, sein.

Die vorliegende Erfindung kann vorteilhaft dazu geeignet und vorgesehen sein, einen in eine Aufnahmeeinrichtung einströmenden Fluidstrom bzw. Fluidschwall zu beruhigen.

Dies kann insbesondere von Vorteil sein, wenn die Aufnahmeeinrichtung einen deutlich größeren Querschnitt aufweist als die Schlauch-, Rohr-, Kanal- oder Ventilquerschnitte, über welche Fluide in die Aufnahmeeinrichtung eingeleitet werden.

Mit der vorliegenden Erfindung kann es vorteilhaft möglich sein, die gewöhnlich beim Durchfließen von Querschnittssprüngen oder -veränderungen auftretenden Turbulenzen und/oder Unregelmäßigkeiten sowohl innerhalb des strömenden Volumens als auch an einer freien Oberfläche (z.B. einem Pegel) zu einem gasgefüllten Raum oder an den fluidbenetzten Oberflächen der Kanäle, Aufnahmeeinrichtung und/oder weiteren Einbauten und dergleichen zu vermeiden.

Beim Ein- oder Ausströmen von Fluiden bei Befüll- und/oder Entleerungsvorgängen gewöhnlich erzeugte Turbulenzen können mittels der vorliegenden Erfindung ebenfalls vorteilhaft verringert oder gar vollständig vermieden werden.

Mit der vorliegenden Erfindung kann es vorteilhaft möglich sein, ein Einrühren, Einbringen oder Verkapseln von Gasblasen, wie beispielsweise Luftblasen, und/oder eine Schaumbildung in den in der Aufnahmeeinrichtung einströmenden oder bereits befindlichen Fluiden zu vermeiden. Insbesondere können vorteilhaft Lufteinschlüsse in den Phasen der Be- und Entnetzung der Seitenwände der Aufnahmeeinrichtung verringert oder vermieden werden.

In der erfindungsgemäßen Aufnahmeeinrichtung können durch Aufteilen des in die Aufnahmeeinrichtung eintretenden Fluidschwalls schwappende und/oder schäumende Pegelflächen der Fluide sowie Prallströmungen und/oder unerwünschte Schwapp- und/oder Rotationsströmungen vorteilhaft verringert oder vermieden werden.

Aufgrund der geringen oder ausbleibenden Bildung von Schwappungen, Wellen und/oder Strudeln in der Flüssigkeitsoberfläche können ein weiterer Gaskontakt und/oder eine weitere Gasaufnahme vermieden werden und beispielsweise ein präzises Messen der Füllhöhe der Flüssigkeit ermöglicht werden.

Anders als bei herkömmlichen strömungsberuhigenden Einbauten oder Formgebungen für Aufnahmeeinrichtungen, kann bei der vorliegenden Erfindung eine Beeinträchtigung der Wirkungsmöglichkeit der Sensorik vorteilhaft vermieden werden.

Dies kann beispielsweise vorteilhaft dazu beitragen, ausreichend genaue Messungen an den in der Aufnahmeeinrichtung befindlichen Fluiden, wie beispielsweise eine möglichst genaue Messung des Füllstands der Fluide, vornehmen zu können.

Ferner kann - beispielsweise bei einer Messung einer Eigenschaft der in der Aufnahmeeinrichtung befindlichen Fluide - die Gefahr bzw. das Problem vermieden werden, durch das Auftreten von Gasblasen bzw. Luftblasen oder Verwirbelungen hervorgerufene verfälschte Messergebnisse zu erhalten.

Im Gegensatz zu großbauenden Vorrichtungen bzw. Einrichtungen aus dem Stand der Technik, wie beispielsweise einer Diffusorform der Kanäle - welche in vielen Fällen Diffusorlängen von mehr als dem Zehnfachen des Kanalquerschnitts erfordert - einer Tauchrohrform des Einströmungskanals bzw. Ausströmungskanals, einer Behälterwanddüse und/oder Prallwänden, kann die vorliegende Erfindung als minimalinvasive, kleinbauende und nicht störend angeordnete Aufnahmeeinrichtung in einer externen Funktionseinrichtung vorgesehen sein.

Das Fluidschwallumleitungselement kann vorteilhaft aus nur einer für ihre Funktion erforderlichen Komponente, dem Umströmungskörper, bestehen.

In vielen Fällen kann das Fluidschwallumleitungselement keine separate Komponente, sondern vorteilhaft eine mehrkosten-, montage-, fugen- und/oder totraumfreie geometrische Ausgestaltung als in eine Wand des Einströmungskanals und/oder eine Seitenwand der Aufnahmeeinrichtung integrierte Geometrie darstellen.

Der Umströmungskörper kann vorteilhaft auf einfache und/oder kostengünstige Weise spritzgießtechnisch realisiert werden, ohne zusätzliche Schieber und/oder Formkerne mit Durchtrittsstellen durch den Formeinsatz mit späterer Fluidberührung zu erfordern. Hierdurch kann vorteilhaft verhindert werden, dass Schmutzstoffe aus den beweglichen Nahtstellen eines Spritzgusswerkzeugs in die fluidbenetzte Oberfläche der Aufnahmeeinrichtung eingebracht werden.

Der Umströmungskörper kann vorteilhaft bei geringem Bedarf an benötigter Mehrbauhöhe, Mehrvolumen und/oder Mehroberfläche - im Vergleich zu einer Aufnahmeeinrichtung ohne strömungsberuhigende Einbauten oder mit anderen strömungsberuhigenden Einbauten als dem erfindungsgemäßen Umströmungskörper - realisiert werden.

Die geringe Oberfläche und/oder die strömungsgünstige Formgebung des Umströmungskörpers können weiter vorteilhaft zu einer Strömungsberuhigung mit minimierter Lufteinbringung, Luftanhaftung, Scherrate und/oder Strömungstoträumen führen.

Der Umströmungskörper kann ferner gerade auch in der Be- und Entnetzungsphase, in welcher der Flüssigkeitspegel gerade noch den Umströmungskörper erfasst oder freigibt, Vorteile mit sich bringen. So kann der Umströmungskörper beispielsweise beim Ablaufen der Fluide vorteilhaft nur eine geringe und reproduzierbare Menge an Fluiden zur Anhaftung an dem Umströmungskörper zurücklassen, was sich in vorteilhafter Weise auf Pegelmessungen und auf das fluidische Verfahren (vollständiger Fluidaustausch) auswirken kann.

Die geringe Oberfläche des Umströmungskörpers, das Nichtvorliegen von scharfen Kanten und/oder Hohlräumen und/oder die geringe Turbulenzen- und/oder Schaumbildung können vorteilhaft zu einer hohen Blutkompatibilität des Umströmungskörpers führen.

Der Umströmungskörper kann bei geringem Raumbedarf dicht vor dem zum Behältnis führenden Einströmungs- und/oder Ausströmungskanal angeordnet sein. In vorteilhafter Weise können Messeinrichtungen, beispielsweise zur Erfassung von Pegeln, von Schaum, von Phasengrenzen und/oder von Luftblasen, störungs- und/oder einbautenfrei in dem darüberliegenden Pegelraum der Aufnahmeeinrichtung angeordnet werden und diesen für die Fluide im Wesentlichen nicht relevanten Raum nutzen.

Im Folgenden wird die vorliegende Erfindung unter Bezugnahme auf die beigefügte Zeichnung beschrieben. In der Zeichnung bezeichnen identische Bezugszeichen gleiche oder identische Elemente. Es zeigt:
- Fig. 1: eine Draufsicht auf die erfindungsgemäße Aufnahmeeinrichtung einer ersten Ausführungsform in einem ersten Zustand;
- Fig. 2: eine Draufsicht auf die erfindungsgemäße Aufnahmeeinrichtung der Fig. 1 in einem zweiten Zustand;
- Fig.3: einen Längsschnitt einer erfindungsgemäßen Aufnahmeeinrichtung;
- Fig. 4: einen Längsschnitt der erfindungsgemäßen Aufnahmeeinrichtung gemäß einer weiteren Ausführungsform;
- Fig. 5: eine Vorderansicht einer an ihrer Vorderseite mit einer Abdeckeinrichtung versehenen erfindungsgemäßen Funktionseinrichtung gemäß einer bevorzugten Ausführungsform;
- Fig. 6: die externe Funktionseinrichtung der Fig. 5 mit nach zerstörendem Aufschneiden aufgeklappter Abdeckeinrichtung; und
- Fig. 7: die externe Funktionseinrichtung aus Fig. 5 und Fig. 6, betrachtet von ihrer Rückseite.

**Fig. 1** zeigt eine erfindungsgemäße Aufnahmeeinrichtung einer ersten Ausführungsform in einer Befüllphase.

Die erfindungsgemäße Aufnahmeeinrichtung 100 weist in ihrem Inneren I ein Fluidschwallumleitungselement auf, welches einen Umströmungskörper 1 aufweist.

Der Umströmungskörper 1 ist innerhalb eines Übergangsbereichs 3 der Aufnahmeeinrichtung 100 zwischen einem Einströmungskanal 5 und einem Nutzbereich 7 der Aufnahmeeinrichtung 100 angeordnet.

Der Übergangsbereich 3 der Aufnahmeeinrichtung 100 ermöglicht einen sinnvollen Übergang zwischen dem relativ kleinen Fluidquerschnitt des Einströmungskanals 5 und dem relativ großen Querschnitt der Aufnahmeeinrichtung 100, wie beispielsweise die Herstellbarkeit der Aufnahmeeinrichtung 100 und des Einströmungskanals 5 aus einem Stück, eine gute Selbstentleerung der Aufnahmeeinrichtung 100 auch bei schräg stehender Anordnung der Aufnahmeeinrichtung 100, die Integrierung eines Kanalverschlussventils direkt am Übergang des Einströmungskanals 5 in die Aufnahmeeinrichtung und dergleichen.

Im Übergangsbereich 3 können beispielsweise Messeinrichtungen zum Erfassen eines Pegelstandes der in der Aufnahmeeinrichtung 100 befindlichen Fluide vorgesehen sein. Derartige Messeinrichtungen können speziell für diese Erfassungszwecke optimiert ausgestaltet sein.

Der Umströmungskörper 1 ist vorzugsweise innerhalb des Übergangsbereichs 3 angeordnet, auch um den Raum des Übergangsbereichs 3 zu nutzen.

Der Nutzbereich 7 der Aufnahmeeinrichtung 100 kann Anforderungen genügen, beispielsweise einem günstigen Verhältnis zwischen Platzbedarf und Füllvolumen der Aufnahmeeinrichtung 100, einer günstigen Messbarkeit des Füllvolumens der Aufnahmeeinrichtung 100 bzw. der darin befindlichen Fluide oder anderer Größen (etwa mittels Transparenz der in der Aufnahmeeinrichtung 100 befindlichen Fluide), einer geringen benetzten Oberfläche im Vergleich zum Füllvolumen, gutes Be- und Entnetzungsverhalten, einem guten Ablaufverhalten der Fluide und dergleichen.

Durch eine Einströmungsöffnung 9 strömt ein Fluidschwall (in Fig. 1 durch einen Blockpfeil gekennzeichnet) in die Aufnahmeeinrichtung 100 ein.

Der in die Aufnahmeeinrichtung 100 einströmende Fluidschwall trifft auf eine Front 11 des Umströmungskörpers 1 auf und wird in zwei Teilfluidschwalle aufgeteilt. Die Teilfluidschwalle werden jeweils an einer rechten Seitenflanke 13 und einer linken Seitenflanke 15 vorbeigeleitet.

Auf einer Rückseite 17 des Umströmungskörpers 1 treffen die Teilfluidschwalle wieder zusammen bzw. aufeinander.

Der Umströmungskörper 1 kann, wie in Fig. 1 gezeigt, einen elliptischen Querschnitt aufweisen.

Der Umströmungskörper 1 ist, wie in Fig. 1 gezeigt, im Wesentlichen symmetrisch in der Einströmungszone des Fluidschwalls angeordnet.

Die Auslegung sowohl der Breite als auch der Höhe (Erstreckung in Strömungsrichtung in Richtung des Doppelpfeils) des Umströmungskörpers 1 können darauf gerichtet sein, dem Umströmungskörper 1 eine in allen Raumrichtungen möglichst kleine Dimension zu geben.
Diese minimal kleinen Dimensionen des Umströmungskörpers 1 können vorzugsweise dazu beitragen, einen möglichst geringen Verlust an nutzbarem Füllvolumen der Aufnahmeeinrichtung 100 sicherzustellen und/oder die zu benetzende und zu entnetzende Oberfläche der Aufnahmeeinrichtung 100 zu verringern.

Eine derartige Oberflächenminimierung kann der erfindungsgemäßen Anordnung der Fluide in mehreren Punkten zugute kommen: Eine geringere Oberfläche kann vorteilhaft zu geringeren Anhaftmöglichkeiten für kleine Gasblasen führen. Eine geringere Oberfläche kann generell die chemische und/oder physikalische Reaktivität der Fluide mit dem Material der Aufnahmeeinrichtung 100 und/oder mit dem in der Aufnahmeeinrichtung befindlichen Gas verringern. Dies kann beispielsweise vorteilhaft zu einer verbesserten Hämokompatibilität und/oder verringerter Gerinnungsneigung einströmenden Bluts durch weniger Luft und weniger Fremdoberfläche führen.

Eine geringere Oberfläche kann die Fluide - im Verhältnis zum maximalen Füllvolumen - in vorteilhafter Weise vollständiger und reproduzierbarer in die Aufnahmeeinrichtung 100 ein- bzw. ausströmen lassen, da geringere Restmengen an dieser Oberfläche haften bleiben können. Die genaue Messung des Füllvolumens anhand des Füllstandes kann erleichtert sein.

Der Austausch von Fluiden zwischen den Befüll-/Entleerungszyklen kann in vorteilhafter Weise vollständiger erfolgen. Die Durchmischung der Fluidchargen kann, was in vielen Fällen verfahrenstechnisch angestrebt sein kann, insbesondere auch im Hinblick auf eine verbesserte Hämokompatibilität durch Vermeidung von Blutanhaftung und Reduktion der Blutverweilzeit an Fremdoberflächen erreicht werden.

Die minimalen Dimensionen des Umströmungskörpers 1 können experimentell ermittelt und so gewählt werden, dass sich der gewünschte Effekt der Beruhigung der Strömungen und der Pegeloberfläche sowie der minimalen Erzeugung und Einrührung von Gas in die in die Aufnahmeeinrichtung 100 einströmenden Fluide gerade noch einstellen kann.

Die minimal möglichen Dimensionen können von der Form und/oder von der mittleren Strömungsgeschwindigkeit im Einströmungskanal 5, von der Zusammensetzung und Zähigkeit der einströmenden Fluide, vom Material und der Oberflächenstruktur der Seitenwände des Einströmungskanals 5, der Aufnahmeeinrichtung 100 und/oder des Umströmungskörpers 1, von der Gestaltung des Übergangsbereichs 3, von der geometrischen Anbindung des Umströmungskörpers 1 an die Aufnahmeeinrichtung 100, von der möglichen Pulsation und dem zulässigen Volumenstrombereich der einströmenden Fluide und/oder von allen geometrischen Einflussfaktoren auf die Strömungen in der näheren und auch etwas weiteren Umgebung (bis etwa zum Zehnfachen des Querschnitts des Einströmungskanals 5) des Umströmungskörpers 1 abhängen.

In vorteilhafter Weise kann der gewünschte Effekt der Strömungsberuhigung bereits bei erstaunlich kleinen Abmessungen des Umströmungskörpers 1 im Bereich von Größen kleiner dem mittleren Strömungsquerschnitt im Einströmungskanal 5 zustande kommen.

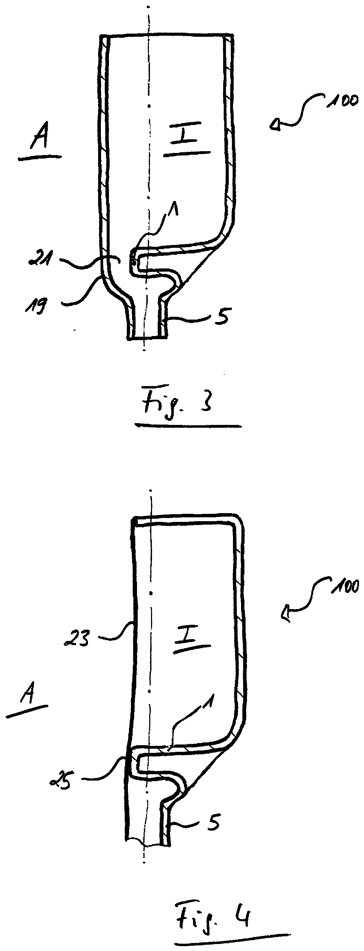

Der Umströmungskörper 1 könnte in alle Dimensionen gekrümmt sein, wie etwa eine Kugel oder ein Ellipsoid. Um die Herstellbarkeit des Umströmungskörpers 1, die fugen- und/oder totraumfreie Anbindung an die Aufnahmeeinrichtung 100 und/oder die preisgünstige Herstellbarkeit aus einem Stück mit der Aufnahmeeinrichtung 100 zu erreichen, kann der Umströmungskörper 1 in besonders bevorzugter Ausführung nur einen in zwei Raumrichtungen gekrümmten Umströmungsquerschnitt aufweisen. Er kann beispielsweise bevorzugt eine im Wesentlichen prismatische Bauform aufweisen, wie sie nachfolgend in Fig. 3 und 4 dargestellt ist.

Durch die Aufteilung des ursprünglich in die Aufnahmeeinrichtung 100 einströmenden Fluidschwalls in wenigstens zwei Teilfluidschwalle kann eine Strömungsberuhigung des Fluidschwalls erfolgen. Der Wirkmechanismus der Strömungsberuhigung kann - im Vergleich zu anderen Lösungen - stärker auf einer gezielten Selbst-Schwallauslöschung beruhen. Er kann insgesamt eine Kombination aus mehreren Strömungseffekten darstellen.

Wie auch in Fig. 1 veranschaulicht, kann eine Verlangsamung der Strömung des Fluidschwalls erzielt werden a) durch Kanalerweiterung des Einströmungskanals 5 zwischen dem Umströmungskörper 1 und den Wandungen bzw. Seitenwänden des Übergangsbereichs 3 und/oder b) durch eine Aufteilung in mindestens zwei gleich und jeweils halb so große entgegengesetzt weiter fließende Teilströmungen oder Teilfluidschwalle und/oder c) durch eine Aufteilung der jeweiligen Teilfluidschwalle in je mindestens zwei weitere Teilfluidschwalle mit entgegengesetztem Impuls bzw. Drall durch Anhaftung an den Umströmungskörper 1 auf der einen Seite und an die jeweilige Seitenwand der Aufnahmeeinrichtung 100 auf der anderen Seite und/oder d) durch ein Aufeinandertreffen und gegenseitige Auslöschung der entgegengesetzten gleichgroßen Strömungs- und Drallimpulse auf der abgewandten Seite, d.h. der Rückseite 17 des Umströmungskörpers 1 und/oder e) durch Aufeinandertreffen und Auslöschung der entgegengesetzt gleichgroßen Strömungsimpulse in den Bereichen zwischen dem Umströmungskörper 1 und einer Seitenwand der Aufnahmeeinrichtung 100 rechts und links des Umströmungskörpers 1 und/oder f) durch Abbau von Strömungsenergie durch Reibungseffekte der vielfachen jeweils entgegengesetzt fließenden und rotierenden Teilströmungen bzw. Teilfluidschwalle untereinander.

Beobachtungen lassen den Schluss zu, dass die erfindungsgemäße Aufnahmeeinrichtung 100 mit der schwallberuhigenden oder -auslöschenden Anordnung sogar einen zusätzlichen beruhigenden Effekt auf anderweitig eingebrachte (etwa durch Stöße an die Aufnahmeeinrichtung) Turbulenzen und/oder Schwappschwingungen der Pegeloberfläche ausüben kann.

Der Umströmungskörper 1 kann einen kreisförmigen, elliptischen, paraboloiden oder gotisch spitzen Querschnitt aufweisen. Der Umströmungskörper 1 kann einen - in Bezug auf die dem Einströmungskanal 5 zu- und abgewandten Seiten - unsymmetrischen Querschnitt aufweisen.

Die symmetrische Ausführung der Geometrie an der rechten Seitenflanke 13 und der linken Seitenflanke 15 kann für die korrekte Funktionsweise der Impulsaufteilungs- und Impulsselbstauslöschungsvorgänge wesentlich sein.

Zur Minimierung des Volumens, des Bauhöhenbedarfs und/oder der Oberfläche des Umströmungskörpers 1 hat sich eine im Wesentlichen querelliptische Form des Umströmungskörpers 1 als vorteilhaft effektiv erwiesen.

In Abhängigkeit von den vorhandenen Fluiden kann es jedoch sinnvoll sein, die Krümmungen auf der dem Einströmungskanal 5 zugewandten Seite des Umströmungskörpers 1 unterschiedlich zu den Krümmungen auf der dem Einströmungskanal 5 abgewandten Seite des Umströmungskörpers 1 auszuführen.

**Fig. 2** zeigt die erfindungsgemäße Aufnahmeeinrichtung 100 in einer Entleerungsphase, in welcher sich die Fluide kurz vor der Ablösung vom Umströmungskörper 1 befinden.

Der Einströmungskanal 5 fungiert in hierbei als Ausströmungskanal.

Wie in Fig. 2 zu sehen ist, kann die rechts-links-symmetrische Ausführung des Umströmungskörpers 1 und/oder der restlichen Aufnahmeeinrichtung 100 in Verbindung mit der allmählichen Erweitung des Strömungsquerschnitts des Ausströmungskanals vor und hinter der engsten Durchtrittzone rechts und links des Umströmungskörpers 1 dazu beitragen, dass die kompakte Zone des Hauptfluids möglichst lange mit den dünnen Fluidzonen der Restbenetzung mit dem Umströmungskörper 1 und/oder mit den Seitenwänden der Aufnahmeeinrichtung 100 in Kontakt bleibt. Dabei wird eine größtmögliche Menge an Restflüssigkeit mit der Hauptströmung mitgezogen, bevor es zur Ablösung und zum Verbleib von Restfilmen auf den Oberflächen des Umströmungskörpers 1 und den Seitenwänden der Aufnahmeeinrichtung 100 kommt.

**Fig. 3** zeigt eine Ausführungsform der erfindungsgemäßen Aufnahmeeinrichtung im Längsschnitt, bei der die Aufnahmeeinrichtung 100, der Einströmungskanal 5 und der Umströmungskörper 1 aus einem Stück spritzgegossen sind.

In der gezeigten Ausführungsform ist der Umströmungskörper 1 stirnseitig nicht mit einer gegenüberliegenden Wandung 19 verbunden, sondern weist einen zur Vermeidung von Strömungstotzonen und/oder Luftnestern hinreichend großen Spalt 21 zur Wandung 19 auf.

**Fig. 4** zeigt eine weitere Ausführungsform der vorliegenden Aufnahmeeinrichtung 100 im Längsschnitt, bei der die Gesamtanordnung in der typischen Kassettenbauform ausgeführt ist.

Bei einer solchen Kassettenbauform können Kanäle, wie beispielsweise ein Einströmungskanal 5, und/oder Kammern in einem Spritzgussteil im Wesentlichen einseitig offen ausgeführt sein. Eine flache und/oder ebenfalls mit halboffenen Strukturen ausgeführte Abdeckeinrichtung 23, beispielsweise ein Deckel oder eine Folie, kann die Aufgabe übernehmen, die Kammern und/oder Kanäle gegen ein Äußeres A abzuschließen. Bei der Ausführung mit Folienverschluss kann die Folie, wie in Fig. 4 gezeigt, per Verschweißung und/oder Verpressung auf eine Stirnfläche 25 des Umströmungskörpers 1 mit direktem Kontakt zu dieser aufgebracht werden. Bei einer solchen Anordnung kann bewusst ein Spalt vermieden werden.

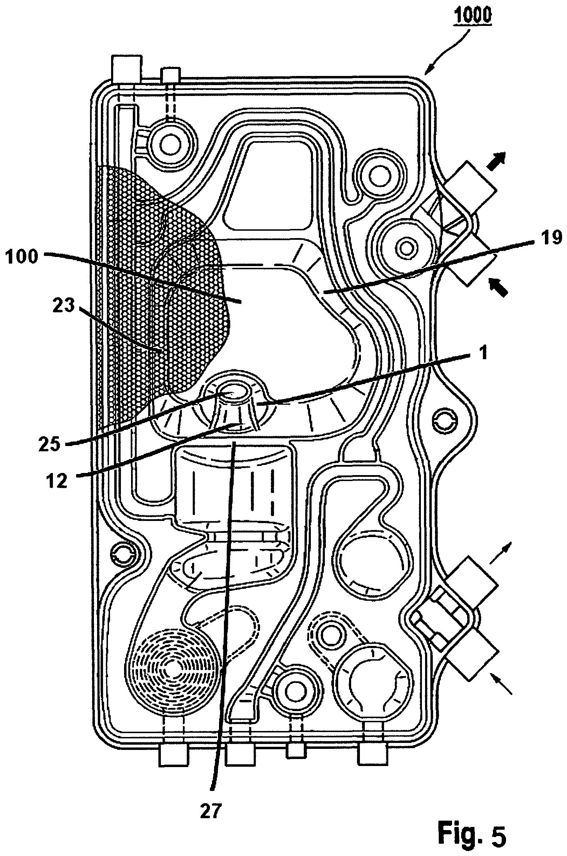

**Fig. 5** zeigt eine Vorderansicht einer erfindungsgemäßen externen Funktionseinrichtung, welche an der Oberfläche, auf die in Fig. 5 geblickt wird, mit einer Abdeckeinrichtung 23 versehen ist.

Die externe Funktionseinrichtung ist hier exemplarisch als Blutbehandlungskassette 1000 ausgestaltet mit Kammern, Kanäle, Ventilen und dergleichen. Die Abdeckeinrichtung 23 deckt eine Vorderseite der Blutbehandlungskassette 1000 ab. Sie ist beispielhaft als Folie ausgestaltet.

Die Blutbehandlungskassette 1000 kann wenigstens mit der in Fig. 5 gezeigten Vorderseite an eine Blutbehandlungsvorrichtung (in Fig. 5 nicht gezeigt) angekoppelt werden.

Die Blutbehandlungskassette 1000 weist die erfindungsgemäße Aufnahmeeinrichtung 100 auf.

Die Aufnahmeeinrichtung 100 erstreckt sich von der in Fig.5 gezeigten Vorderansicht nach hinten in die Zeichenebene der Fig. 5 hinein. Der Verlauf der Wandung 19 der Aufnahmeeinrichtung 100 von dem Betrachter weg ist durch die strahlenförmig gezeigten Linien angedeutet. Der Umströmungskörper 1 erstreckt sich von seiner Basis in Richtung auf den Betrachter der Fig. 1 zu.

Die Aufnahmeeinrichtung 100 weist den Umströmungskörper 1 auf. In Fig.5 sind vom Umströmungskörper 1 besonders gut die Stirnfläche 25 sowie ein in die Zeichenebene tiefer liegender sattelförmiger Verbindungsbereich 12 zwischen dem Umströmungskörper 1 und der Wandung 19 zu erkennen.

Die Aufnahmeeinrichtung 100 weist an ihrem unteren Ende, d.h. am Anströmbereich, ein Phantomventil 27 auf. Der Umströmungskörper 1 wird in der Aufnahmeeinrichtung 100 über das Phantomventil 27 angeströmt, wobei Blut aus der darunter liegenden Kammer (z.B. einer venösen Blutkammer, wie sie in der von der Anmelderin der vorliegenden Anmeldung beim DPMA am 10. Juni 2009 hinterlegten Anmeldung 10 2009 024 465 A1 mit dem Titel *"Luftabscheider, externe Funktionseinrichtung, Blutkreislauf sowie Behandlungsvorrichtung"* beschrieben wurde) durch das von der Abdeckeinrichtung 23 und einen Stegabschnitt gebildete Phantomventil 27 in die Aufnahmeeinrichtung 100 einströmt.

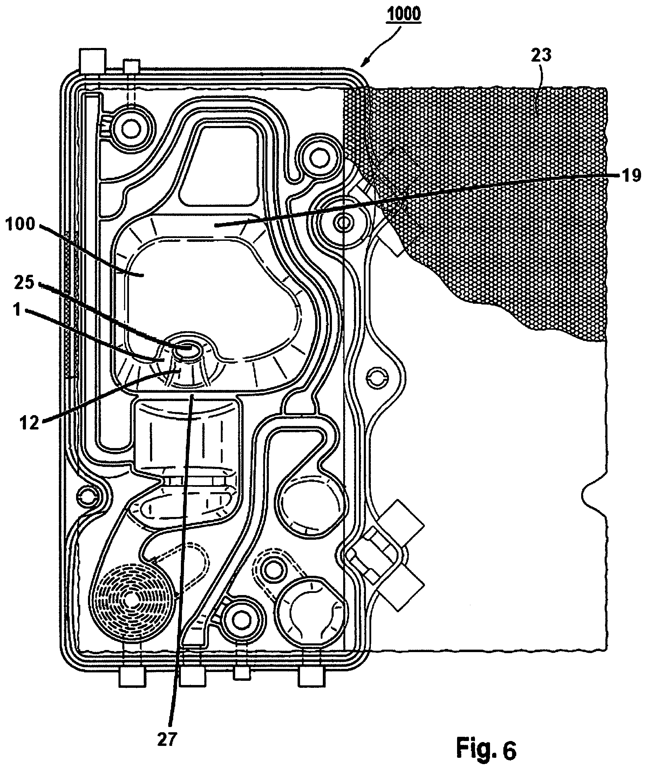

**Fig. 6** zeigt die Blutbehandlungskassette 1000 der Fig. 5, wobei die Abdeckeinrichtung 23 am linken Rand der Blutbehandlungskassette 1000 sowie oben und unten zustörend aufgeschnitten und nach rechts aufgeklappt zu erkennen ist.

Fig. 6 zeigt die nach Aufschneiden der Folie detaillierter zu erkennenden Elemente im Inneren der Blutbehandlungskassette 1000.

Zur Vermeidung von Wiederholungen wird auf die vorstehend bei der Beschreibung der Fig. 5 erörterten Ausgestaltungen der einzelnen Elemente verwiesen.

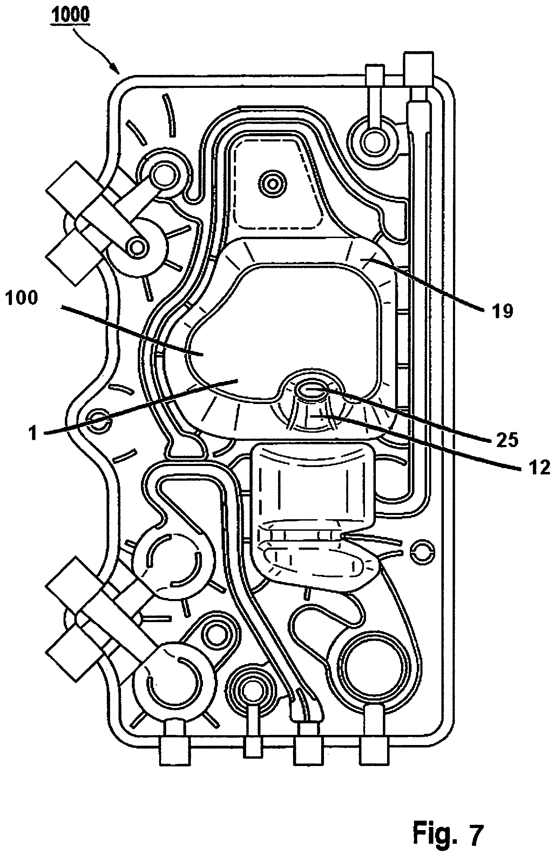

**Fig. 7** zeigt die Blutbehandlungskassette 1000 von ihrer Rückseite. Ist die Blutbehandlungskassette 1000 an die Blutbehandlungsvorrichtung angekoppelt, so wird ein Betrachter beim Öffnen einer Tür der Blutbehandlungsvorrichtung zum Entnehmen der Blutbehandlungskassette 1000 auf diese Rückseite blicken.

Für weitere Details zur Blutbehandlungskassette 1000 wird auf deren ausführliche Beschreibung in der hierauf gerichteten, o.g. Anmeldung verwiesen.

## Patentansprüche

1. Aufnahmeeinrichtung (100) zum Aufnehmen von medizinischen Fluiden, insbesondere Blut, mit
- wenigstens einer Einströmungsöffnung (5), durch welche wenigstens ein Fluid im Schwall in ein Inneres (I) der Aufnahmeeinrichtung (100) einbringbar ist,
- wenigstens ein Fluidschwallumleitungselement zum Umleiten des Fluidschwalls des einströmenden Fluids, wobei
- das Fluidschwallumleitungselement wenigstens einen Umströmungskörper (1) zum Aufteilen des Fluidschwalls des einströmenden Fluids in wenigstens zwei Teilfluidschwalle aufweist,
**dadurch gekennzeichnet, dass**
- der Umströmungskörper im Ausströmungsbereich eines Phantomventils in einer Blutkassette zur extrakorporalen Blutbehandlung und in einem Einströmungsbereich des Fluidschwalls in der Aufnahmeeinrichtung angeordnet ist, wobei das Phantomventil ein Element mit einer mittels Aktor erreichbaren Aktor-Fläche umfasst, wobei das Element die Funktion eines Ventils übernehmen kann.

2. Aufnahmeeinrichtung nach Anspruch 1, wobei der Umströmungskörper (1) symmetrisch ausgestaltet ist.

3. Aufnahmeeinrichtung nach Anspruch 1, wobei der Umströmungskörper (1) einen kreisförmigen Querschnitt aufweist.

4. Aufnahmeeinrichtung nach Anspruch 1, wobei der Umströmungskörper (1) einen elliptischen Querschnitt aufweist.

5. Aufnahmeeinrichtung nach Anspruch 1, wobei der Umströmungskörper (1) einen paraboloiden Querschnitt aufweist.

6. Aufnahmeeinrichtung nach einem der vorangegangenen Ansprüche, wobei der Umströmungskörper (1) in einem Abstand des 0,4fachen bis 2fachen des Durchmessers der Einströmungsöffnung von dieser entfernt im Einströmungsbereich der Aufnahmeeinrichtung (100) angeordnet ist.

7. Aufnahmeeinrichtung nach einem der vorangegangenen Ansprüche, wobei ein Durchmesser des Umströmungskörpers (1) in einer Ebene senkrecht zu einer Mittelachse der Einströmungsöffnung 0,4 bis 1,2-mal so groß wie der Durchmesser der Einströmungsöffnung ist.

8. Aufnahmeeinrichtung nach einem der vorangegangenen Ansprüche, wobei das Fluidschwallumleitungselement stoffschlüssig mit der Aufnahmeeinrichtung (100) ausgestaltet ist.

9. Aufnahmeeinrichtung nach Anspruch 8, wobei die Aufnahmeeinrichtung (100) mittels Spritzguss ausgestaltet ist.

10. Aufnahmeeinrichtung nach Anspruch 8, wobei die Aufnahmeeinrichtung (100) stoffschlüssig in eine externe Funktionseinrichtung integriert ist.

11. Aufnahmeeinrichtung nach Anspruch 10, wobei die externe Funktionseinrichtung an wenigstens einer Seite mit einem Abdeckungselement (23) versehen ist.

12. Aufnahmeeinrichtung nach Anspruch 11, wobei das Abdeckungselement (23) auf einer Oberseite des Umströmungskörpers (1) aufliegt.

13. Aufnahmeeinrichtung nach einem der vorangegangenen Ansprüche, welche in einem extrakorporalen Blutkreislauf angeordnet ist.

14. Externe Funktionseinrichtung zum Behandeln wenigstens eines Fluids, welche eine Aufnahmeeinrichtung (100) gemäß einem der Ansprüche 1 bis 13 aufweist und als Blutkassette und/oder als Spritzguss-Hartteil mit einer aufgeschweißten Folie ausgestaltet ist.

15. Medizinische Behandlungsvorrichtung zum Behandeln wenigstens eines Fluids, welche eine Aufnahmeeinrichtung (100) gemäß einem der Ansprüche 1 bis 13 oder eine externe Funktionseinrichtung gemäß dem Anspruch 14 aufweist und die medizinische Behandlungsvorrichtung eine Blutbehandlungsvorrichtung ist.

## Claims

1. A receiving device (100) for receiving medical fluids, in particular blood, comprising:
- at least one inflow opening (5) through which at least one fluid in surge can be introduced into an interior (I) of the receiving device (100),
- at least one fluid surge redirecting element for redirecting the fluid surge of the inflowing fluid,
- wherein the fluid surge redirecting element comprises at least one flow-conditioning body (1) for dividing the fluid surge of the inflowing fluid into at least two partial fluid surges,
**characterized in that**:
- the flow-conditioning body is arranged in the outflow region of a phantom valve in a blood cassette for extracorporeal blood treatment and in an inflow region of the fluid surge in the receiving device,
- wherein the phantom valve comprises an element with an actuator surface reachable by means of an actuator, the element being able to assume the function of a valve.

2. The receiving device according to claim 1, wherein the flow-conditioning body (1) is symmetrically configured.

3. The receiving device according to claim 1, wherein the flow-conditioning body (1) comprises a circular cross-section.

4. The receiving device according to claim 1, wherein the flow-conditioning body (1) comprises an elliptical cross-section.

5. The receiving device according to claim 1, wherein the flow-conditioning body (1) comprises a paraboloidal cross-section.

6. The receiving device according to anyone of the preceding claims, wherein the flow-conditioning body (1) is arranged in the inflow region of the receiving device (100) at a distance of 0.4 to 2 times the diameter of the inflow opening from the inflow opening.

7. The receiving device according to anyone of the preceding claims, wherein a diameter of the flow-conditioning body (1) is 0.4 to 1.2 times as big as the diameter of the inflow opening in a plane perpendicular to a center axis of the inflow opening.

8. The receiving device according to anyone of the preceding claims, wherein the fluid surge redirecting element is realized in material connection with the receiving device (100).

9. The receiving device according to claim 8, wherein the receiving device (100) is realized by means of injection molding.

10. The receiving device according to claim 8, wherein the receiving device (100) is integrated into an external functional device by material connection.

11. The receiving device according to claim 10, wherein the external functional device is provided, on at least one side, with a cover element (23).

12. The receiving device according to claim 11, wherein the cover element (23) rests on an upper side of the flow-conditioning body (1).

13. The receiving device according to anyone of the preceding claims, which is arranged in an extracorporeal blood circuit.

14. An external functional device for treating at least one fluid, which comprises a receiving device (100) according to anyone of claims 1 to 13 and which is configured as a blood cassette and/or as an injection-moulded hard part with a welded-on film.

15. A medical treatment apparatus for treating at least one fluid, comprising a receiving device (100) according to anyone of claims 1 to 13 or an external functional device according to claim 14, wherein the medical treatment apparatus is a blood treatment apparatus.

## Revendications

1. Un dispositif de réception (100) pour recevoir des fluides médicaux, en particulier du sang, comprenant :
- au moins un orifice d'entrée (5) par lequel au moins une projection de fluide peut être introduite à l'intérieur (I) du dispositif de réception (100),
- au moins un élément de déviation de projection de fluide pour dévier la projection de fluide du fluide entrant,
- où l'élément de déviation de projection de fluide comprend au moins un corps de contournement d'écoulement (1) pour répartir la projection de fluide du fluide entrant en au moins deux projections de fluide partielles,
**caractérisé en ce que**:
- le corps de contournement d'écoulement est agencé dans la zone de sortie d'une vanne fantôme dans une cassette à sang prévue pour un traitement sanguin extracorporel et dans une zone d'entrée de la projection de fluide dans le dispositif de réception,
- où la vanne fantôme comprend un élément avec une surface d'actionnement accessible au moyen d'un actionneur, l'élément pouvant assumer la fonction d'une vanne.

2. Le dispositif de réception selon la revendication 1, où le corps de contournement d'écoulement (1) est conçu de façon symétrique.

3. Le dispositif de réception selon la revendication 1, où le corps de contournement d'écoulement (1) comprend une portion transversale circulaire.

4. Le dispositif de réception selon la revendication 1, où le corps de contournement d'écoulement (1) comprend une portion transversale elliptique.

5. Le dispositif de réception selon la revendication 1, où le corps de contournement d'écoulement (1) comprend une portion transversale paraboloïdale.

6. Le dispositif de réception selon l'une quelconque des revendications précédentes, où le corps de contournement d'écoulement (1) est agencé dans la zone d'entrée du dispositif de réception (100) à une distance de 0,4 à 2 fois le diamètre de l'orifice d'entrée de celui-ci.

7. Le dispositif de réception selon l'une quelconque des revendications précédentes, où un diamètre du corps de contournement d'écoulement (1) est de 0,4 à 1,2 fois plus grand que le diamètre de l'orifice d'entrée) dans un plan perpendiculaire à un axe central de l'orifice d'entrée.

8. Le dispositif de réception selon l'une quelconque des revendications précédentes, où l'élément de déviation de projection de fluide est conçu de manière adhérente avec le dispositif de réception (100).

9. Le dispositif de réception selon la revendication 8, où le dispositif de réception (100) est conçu au moyen d'un moulage par injection.

10. Le dispositif de réception selon la revendication 8, où le dispositif de réception (100) est intégré de manière adhérente à un dispositif de fonction externe.

11. Le dispositif de réception selon la revendication 10, où le dispositif de fonction externe est doté, sur au moins un côté, d'un élément de recouvrement (23).

12. Le dispositif de réception selon la revendication 11, où l'élément de recouvrement (23) repose sur une face supérieure du corps de contournement d'écoulement (1).

13. Le dispositif de réception selon l'une quelconque des revendications précédentes, qui est agencé dans un circuit sanguin extracorporel.

14. Un dispositif de fonction externe pour traiter au moins un fluide comprenant un dispositif de réception (100) selon l'une quelconque des revendications 1 à 13 et conçu comme une cassette à sang et/ou comme une pièce dure moulée par injection avec un film soudé.

15. Un appareil de traitement médical pour traiter au moins un fluide comprenant un dispositif de réception (100) selon l'une quelconque des revendications 1 à 13 ou un dispositif de fonction externe selon la revendication 14 et où l'appareil de traitement médical est un appareil de traitement du sang.
